# EUROPÄISCHE PATENTANMELDUNG

(11) **EP 3 604 844 A1**
(43) Veröffentlichungstag der Anmeldung: **05.02.2020**
(21) Anmeldenummer: 18187397.7
(22) Anmeldetag: 03.08.2018
(51) Int. Cl.: F16C 41/00, F16C 33/58, F16D 65/18

(54) **LAGERANORDNUNG**

(71) Anmelder: Ondal Medical Systems GmbH, 36088 Hünfeld (DE)
(72) Erfinder: Stroop, Nicolas, 33602 Bielefeld (DE)
(74) Vertreter: Graf von Stosch Patentanwaltsgesellschaft mbH

(57) **Zusammenfassung**

Die Erfindung betrifft Lageranordnung (10) mit wenigstens einem ersten Lagerelement (12) und einem zweiten Lagerelement (14), welche entlang einer gemeinsamen Längsachse (16) relativ rotierbar zueinander miteinander verbunden sind, wobei die Lageranordnung (10) eine Bremsvorrichtung (18) umfasst, welche die relative Rotation der beiden Lagerelemente (12, 14) zueinander mittels einer durch die Bremsvorrichtung (18) erzeugten Reibkraft hemmt, und eine Verstellvorrichtung (22), mittels welcher die bei der relativen Rotation der beiden Lagerelemente (12, 14) zueinander wirkende Bremskraft modifizierbar ist, wobei die Verstellvorrichtung (22) wenigstens zwei aneinander an ihrer jeweiligen Keilfläche (34, 36) anliegende Keilelemente (28, 32) umfasst, welche zum Modifizieren der Bremskraft aneinander abgleitend relativ zueinander bewegbar sind.

## Beschreibung

Die Erfindung betrifft eine Lageranordnung mit wenigstens einem ersten Lagerelement und einem zweiten Lagerelement.

Bei bekannten elektromagnetisch betätigten Lagereinheiten wird eine Ankerplatte mit einem aufgebrachten Reibbelag durch vorgespannte Druckfedern gegen eine Gegenfläche, beispielsweise eine Druckplatte, gepresst, um eine Bewegung jeweiliger Lagerelemente zueinander zu hemmen. Die Ankerplatte ist rotationsfest mit einem ersten Gehäuseteil verbunden, welches als Stator bezeichnet werden kann. Die Druckplatte ist rotationsfest mit einem zweiten Gehäuseteil verbunden, welches nachfolgend als Rotor bezeichnet werden kann. Der Reibbelag kann dabei sowohl an der Ankerplatte, als auch an der Druckplatte angebracht sein.

In einem gebremsten Zustand erzeugt die durch die Anpresskraft der Druckfedern erzeugte Reibung ein Bremsmoment. Das Bremsmoment verhindert beispielsweise eine unbeabsichtigte Bewegung eines Tragarmsystems, welches mittels der Lagereinheit wenigstens bezüglich eines rotatorischen Freiheitsgrad beweglich gehalten ist. Bei einer elektromagnetischen Betätigung der Lagereinheit, welche auch als Aktivierung bezeichnet werden kann, wird ein starker Elektromagnet, welcher beispielsweise fest mit dem Stator verbunden ist, mit einem Strom beaufschlagt. Das entstehende Magnetfeld wirkt der Druckkraft der Federn entgegen und zieht die Ankerplatte an. Das Anpressen des Reibbelags wird dadurch beendet und Stator und Rotor lassen sich dann mit geringem Kraftaufwand gegeneinander verdrehen. Charakteristisch bei diesem Wirkprinzip ist, dass die Anpresskraft, welche das Bremsmoment verursacht, der Kraft entspricht, welcher der Elektromagnet zum Lüften der Bremse erzeugen muss. Bei einem hohen Bremsmoment muss der Elektromagnet also entsprechend stark dimensioniert sein und die Ströme innerhalb einer Spule des Elektromagneten sind entsprechend groß. Ebenso ist der Spalt zwischen Elektromagnet und Ankerplatte für die Funktion bei hohen Bremsmomenten ausschlaggebend, da die magnetische Kraft mit dem Abstand zum Magneten stark abnimmt. Dies stellt hohe Anforderungen an die Fertigungsgenauigkeit und den Montageaufwand, insbesondere um den Spalt gering zu halten. Beispielsweise wird zur Einstellung des Spalts ein aufwendig zu fertigendes Feingewinde mit geringer Steigung zwischen Druckplatte und Gehäuse vorgesehen. Die Lage der Druckplatte am Feingewinde wird dabei nach Positionseinstellung mittels eines Klebstoffes fixiert, was eine Wartung sehr aufwendig macht.

Eine weitere bekannte konstruktive Lösung zum Festsetzen oder Bremsen einer Lagereinheit ist die Verwendung eines elastischen Druckschlauches, welcher durch eine Beaufschlagung mit einem pneumatischen Innendruck expandiert. Die Expansion führt zu einer Anpresskraft auf Rotor und Stator und erzeugt an den Wirkflächen eine Reibkraft, welche die Drehbewegung hemmt. Der Druckschlauch kann also beispielsweise die Funktion der Federn und des Elektromagneten übernehmen. Nachteilig bei dieser Lösung ist, dass bei Bewegung eines mittels der Lagereinheit gelagerten Tragarmsystems bei ungelöster Bremse ein abrasiver Verschleiß an den relativ bewegten Wirkflächen des Druckschlauchs, welcher auch als Pneumatikschlauch bezeichnet wird, auftritt. Dieser abrasive Verschleiß kann zu einer Undichtigkeit des Druckschlauchs, welcher auch als Bremsschlauch bezeichnet wird, führen. Die Lebensdauer der Lagereinheit kann somit gering sein und/oder jeweilige Wartungsintervalle können kurz sein. Ein ähnlicher Effekt kann durch Risse an jeweiligen Crimpstellen des elastischen Schlauches, an welchen dieser befestigt ist, auftreten. Die Wartung eines defekten Bremsschlauchs ist ein aufwendiger Serviceeinsatz.

Eine dritte bekannte konstruktive Lösung zum Festsetzen oder Bremsen einer Lagereinheit ist eine Friktionsbremseinheit, welche nicht lösbar ist. Hierbei wird ein dauerhaftes Bremsmoment zwischen Rotor und Stator erzeugt, welches vom Bediener nicht gelöst werden kann. Üblicherweise ist das Bremsmoment dabei so hoch gewählt, dass eine Bewegung eines mit der Lagereinheit gelagerten Tragarmsystems dennoch möglich ist, ungewollte Bewegungen aber relativ zuverlässig verhindert werden. Bei einigen Ausführungsformen ist dieses Bremsmoment nicht einstellbar, ändert sich mit fortschreitendem Verschleiß der Reibflächen und ist dann nicht mehr korrigierbar.

Den beschriebenen Lagereinheiten ist zudem gemein, dass sich diese durch eine hohe Variantenvielfalt von Bauteilen und Baugruppen auszeichnen. Auch dadurch sind diese Lagereinheiten teuer und aufwendig zu warten.

Aus der DE 10 2007 003 470 A1 ist eine Vorrichtung zur Steuerung des Reibmoments eines Lagers bekannt, wobei diese Vorrichtung in einem Lager eingebaut ist. Diese Vorrichtung ist jedoch vergleichsweise groß und fordert einen hohen Kraftaufwand zum Einstellen der Bremskraft.

Aufgabe der vorliegenden Erfindung ist es, eine Lageranordnung zur Verfügung zu stellen, bei welcher eine Reibkraft mit einer bauraumsparenden Vorrichtung und geringem Kraftaufwand eingestellt werden kann.

Diese Aufgabe wird durch den Gegenstand des unabhängigen Patentanspruchs gelöst. Vorteilhafte Ausgestaltung der zweckmäßigen Weiterbildung der Erfindung sind in den jeweiligen Unteransprüchen angegeben.

Die Erfindung betrifft eine Lageranordnung. Die Lageranordnung kann wenigstens ein erstes Lagerelement und ein zweites Lagerelement umfassen, welche entlang einer gemeinsamen Längsachse relativ rotierbar zueinander miteinander verbunden sind. Die Lageranordnung kann eine Bremsvorrichtung umfassen, welche die relative Rotation der beiden Lagerelemente zueinander mittels einer durch die Bremsvorrichtung erzeugten Reibkraft hemmt. Weiterhin kann die Lageranordnung eine Verstellvorrichtung umfassen, mittels welcher die bei der relativen Rotation der beiden Lagerelemente zueinander wirkende Bremskraft modifizierbar ist. Die Verstellvorrichtung kann wenigstens zwei aneinander an ihren jeweiligen Keilflächen anliegende Keilelemente umfassen, welche zum Modifizieren der Bremskraft aneinander abgleitend relativ zueinander bewegbar sind.

Die Keilelemente ermöglichen es dabei, eine hohe Übersetzung bei gleichzeitig geringem Bauraum zur Verfügung zu stellen. Dadurch kann die die Rotation hemmende Reibkraft besonders einfach eingestellt werden. Beispielsweise kann bei einer hohen Übersetzung bzw. Untersetzung mit einer geringen Aktuation der Verstellvorrichtung die Reibkraft über einen hohen Bereich und/oder mit einem geringen Kraftaufwand eine Vorspannung eines Bremsflächenpaars zum Erhöhen der Reibkraft eingestellt werden. Die Keilelemente können dabei konstruktiv sehr simple Bauteile sein, und dabei zudem sehr robust sein und somit hohe Lasten aufnehmen. Dadurch kann die Verstellvorrichtung und damit die Lageranordnung sehr kompakt und robust sein. Insbesondere kann die Verstellvorrichtung mit der mittels jeweiliger Keilelemente bereitgestellten Übersetzung sehr verschleißarm sein.

Die Lageranordnung ist beispielsweise besonders geeignet dafür, jeweilige Tragarme von Gerätehaltern zum Halten medizinischer Geräte bewegbar zu befestigen. Beispielsweise kann mit der Lageranordnung ein Tragarm von OP-Geräten an einer Decke eines OP-Saals befestigt werden. Mittels der Bremsvorrichtung kann dabei verhindert werden, dass sich der Tragarm bei einer versehentlichen Berührung ungewollt bewegt. Gleichzeitig kann aufgrund der Verstellvorrichtung die Bremskraft so eingestellt werden, dass die beiden Lagerelemente und damit ein Tragarm noch, insbesondere ohne zu großen, Kraftaufwand von einem Bediener manuell bewegt werden können.

Die Reibkraft kann beispielsweise dadurch erzeugt werden, dass die Bremsvorrichtung zwei Reibflächen aneinanderpresst, wobei diese Reibflächen bei einer relativen Rotation der beiden Lagerelemente zueinander relativ zueinander bewegt werden. Dadurch wird eine Reibkraft zwischen den beiden Reibflächen erzeugt, welche in einer Rückkoppelung die Bewegung hemmen kann. Die Pressung kann dabei so hoch sein, dass die Reibkraft im Wesentlichen die beiden Lagerelemente aneinander fixiert und eine Bewegung gänzlich verhindert. Vorzugsweise ist die Reibkraft jedoch so hoch, dass die beiden Lagerelemente immer noch relativ zueinander bewegt werden können. Die die Bewegung hemmende Reibkraft kann auch als Bremskraft bezeichnet werden. Die Lageranordnung kann auch als Lageranordnung mit Friktionsbremseinheit oder integrierter Bremsfunktion bezeichnet werden. Insbesondere kann die Bremsvorrichtung mittels der Verstellvorrichtung schaltbar und/oder die bei der relativen Bewegung der Lagerelemente zueinander wirkende Reibkraft einstellbar sein. Mittels der Verstellvorrichtung kann beispielsweise die Bremsvorrichtung aus einem Kraftfluss ausgekuppelt werden, so dass bei einer relativen Rotation der beiden Lagerelemente zueinander Reibkraft nicht mehr die Bewegung hemmt. Die Verstellvorrichtung kann in diesem Fall also als schaltbare Kupplung fungieren. Bei dem ausgekuppelten Zustand wirkt beispielsweise nur noch eine Reibung aufgrund der jeweiligen Lagerung der beiden Lagerelemente aneinander, beispielsweise durch ein Abrollen und/oder Gleiten von jeweiligen Kugeln eines Kugellagers bzw. Wälzlagers. Die Bremskraft bzw. Reibkraft wird dann dahingehend modifiziert, dass diese ein- oder ausgeschaltet werden kann. Modifizieren kann hier deswegen ein Lösen / vollständiges Entkuppeln bzw. Aktivieren / Einkuppeln der Bremse bedeuten.

Alternativ oder zusätzlich kann auch ein Betrag der Reibkraft bei einer relativen Bewegung der Lagerelemente zueinander mittels der Verstellvorrichtung verändert werden. Durch das relative Bewegen der Keilelemente zueinander können dabei jeweilige Reibflächenpaare stärker oder weniger stark gegeneinander gepresst werden. Die Modifikation der Reibkraft betrifft dann in diesem Falle die Stärke der Bewegungshemmung. Die Reibkraft ist in diesem Falle einstellbar. Eine einstellbare Reibkraft kann dabei auch dahingehend schaltbar sein, als diese so weit reduziert werden kann, dass diese im Wesentlichen null entspricht.

In weiterer vorteilhafter Ausgestaltung der Lageranordnung ist es vorgesehen, dass die Bremsvorrichtung eine erste Reibfläche aufweist, welche mit dem ersten Lagerelement um die Längsachse rotationsfest mitrotiert und eine zweite Reibfläche, welche mit dem zweiten Lagerelement um die Längsachse rotationsfest mitrotiert, wobei die beiden Reibflächen gegeneinander mit einer Vorspannkraft verspannt sind und bei der relativen Rotation der beiden Lagerelemente zueinander aneinander unter Erzeugung der Reibkraft abgleiten, wobei die Verstellvorrichtung dazu ausgebildet ist, diese Vorspannkraft durch Bewegung der Keilelemente relativ zueinander zum Einstellen der Reibkraft einzustellen und somit die Bremskraft zu modifizieren. Durch die Verstellvorrichtung können also die beiden Reibflächen stärker oder weniger stark gegeneinandergepresst werden. Beispielsweise kann eine der beiden Reibflächen auf einem der beiden Keilelemente angeordnet sein, welches dann durch ein Bewegen der beiden Keilelemente relativ zueinander stärker oder weniger stark gegen eines der beiden Lagerelemente gepresst wird. Vorzugsweise ist dieses Keilelement oder die Verstellvorrichtung insgesamt dann mit dem anderen der beiden Lagerelemente rotationsfest verbunden. Aufgrund der hohen möglichen Übersetzung und der hohen möglichen Lastaufnahme durch die jeweiligen Keilelemente kann so mit geringem Bauraum eine große Pressung erreicht werden, so dass auch bei geringen zur Verfügung stehenden Reibflächen eine hohe Reibkraft zuverlässig zur Verfügung gestellt werden kann. Die Lageranordnung ist so besonders kompakt und/oder kann so eine besonders hohe Bandbreite an Stärke der Rotationshemmung zur Verfügung stellen. Rotationsfest kann hier bedeuten, dass ein Teil mit einem der Lagerelemente bei dessen Rotation um die Längsachse mitbewegt wird bzw. wenn das zugeordnete Lagerelement nicht um die Längsachse rotiert, dass auch dieses Teil nicht rotiert.

Die Vorspannkraft presst die jeweiligen Reibflächen vorzugsweise in einer Richtung orthogonal zu ihren jeweiligen Flächen aufeinander. Die beiden Reibflächen sind dabei vorzugsweise parallel zueinander ausgerichtet. Ein Aufeinanderzubewegen der beiden Keilelemente kann dabei vorzugsweise die Vorspannkraft verringern und ein Wegbewegen der beiden Keilelemente voneinander erhöht vorzugsweise die Vorspannkraft. Proportional zur Vorspannkraft ist damit die Reibkraft hinsichtlich ihrer Größe einstellbar.

In weiterer vorteilhafter Ausgestaltung der Lageranordnung ist vorgesehen, dass die Lageranordnung eine Kupplungsvorrichtung, welche zwischen einem offenen Zustand und einem geschlossenen Zustand verstellbar ist, umfasst, wobei in dem geschlossenen Zustand die Bremsvorrichtung eingekuppelt ist, wodurch bei einer Rotation der beiden Lagerelemente relativ zueinander diese Rotation durch die mittels der Bremsvorrichtung erzeugte Reibkraft gehemmt ist, und wobei in dem offenen Zustand die Bremsvorrichtung ausgekuppelt ist, wodurch die beiden Lagerelemente frei von einer Hemmung durch die Bremsvorrichtung relativ zueinander rotierbar sind, und wobei die Verstellvorrichtung dazu ausgebildet ist, durch Bewegen der Keilelemente relativ zueinander die Kupplungsvorrichtung zwischen ihrem offenen Zustand und ihrem geschlossenen Zustand zu verstellen und somit die Bremskraft zu modifizieren. Hier kann mit einfachen und bauraumsparenden Mitteln eine schaltbare Friktionseinheit realisiert werden. Hierbei kann es sich nicht nur um eine Reibkraftkupplung handeln, sondern auch um eine Kupplung, bei welcher mittels der Verstellvorrichtung jeweilige formschlüssige Elemente in Eingriff bzw. aus einem Eingriff gebracht werden. Beispielsweise kann mittels der Verstellvorrichtung eine Verzahnung in Eingriff gebracht werden und damit ein formschlüssiger Kraftschluss gebildet werden, so dass die Bremsvorrichtung in den Kraftfluss zwischen dem ersten Lagerelement und dem zweiten Lagerelement eingekuppelt ist. Umgekehrt kann die Verstellvorrichtung diesen Eingriff auch beenden bzw. lösen, sodass die Bremsvorrichtung ausgekuppelt ist.

Fall die Lageranordnung eine Kupplungsvorrichtung umfasst, ist die Bremsvorrichtung vorzugsweise dazu ausgebildet, dauerhaft eine Vorspannkraft zum Erzeugen der Reibkraft zur Verfügung zu stellen. Dadurch kann die Reibkraft unabhängig von deren Aktivierung bzw. Deaktivierung eingestellt werden. Dies kann insbesondere Vorteile hinsichtlich von Fertigungstoleranzen und Fertigungskosten aufweisen. Die Kupplungsvorrichtung und die Bremsvorrichtung können funktional getrennt sein, so dass die Kupplungsvorrichtung einfach und kostengünstig sein kann. Beispielsweise muss die Kupplungsvorrichtung nicht mehr einen Magneten und einen präzise gefertigten Spalt aufweisen, wobei dieser Magnet gleichzeitig die Vorspannkraft zur Erzeugung der Reibkraft bewirkt und/oder stark genug sein muss, die Vorspannkraft aufheben zu können. Die Vorspannkraft kann so unabhängig von der Kupplung und der Kraft zu deren Betätigung sein, und umgekehrt.

Zum Einstellen der Reibkraft separat von der Betätigungskraft der Kupplungsvorrichtung kann die Bremsvorrichtung beispielsweise ein Reibelement und wenigstens eine Spannvorrichtung aufweisen, mittels welcher das Reibelement dauerhaft gegen eines der beiden Lagerelemente verspannt ist. Bei der Verstellvorrichtung können die Keilelemente aneinander abgleiten und dadurch ihren relativen Abstand, insbesondere einen Abstand ihres Mittelpunktes, ändern, wodurch dann ein Kupplungselement in Eingriff mit einem weiteren korrespondierenden Kupplungselement gebracht werden kann oder wodurch ein solcher Eingriff getrennt werden kann.

Ein aufeinander Zubewegen und voneinander Wegbewegen der Keilelemente kann bedeuten, dass sich die Keilelemente insgesamt annähern oder voneinander entfernen. Es muss jedoch nicht bedeuten, dass eine Kontaktfläche zwischen den Keilelementen vergrößert wird oder ein Kontakt zwischen den Keilelementen gelöst wird.

In weiterer vorteilhafter Ausgestaltung der Lageranordnung ist es vorgesehen, dass die Verstellvorrichtung so ausgebildet ist, dass durch das relative Bewegen der Keilelemente zueinander sich diese in einer Richtung im Wesentlichen parallel zu der Längsachse wahlweise aufeinander zubewegen oder voneinander wegbewegen. Dadurch kann die Lageranordnung besonders bauraumsparend sein, insbesondere kann deren radiale Erstreckung besonders gering sein. Eines der beiden Keilelemente kann dabei eine Stirnfläche eines der beiden Lagerelemente bilden oder daran angeordnet sein und gegen eine gegenüberliegende Stirnfläche des anderen der beiden Lagerelemente drücken, um so die Reibkraft bei deren relativen Rotation zueinander bereitzustellen. Vorzugsweise ist dafür dieses Keilelement mit dem Lagerelement, gegen welches es nicht drückt, rotationsfest verbunden.

Die Bewegungsrichtung der jeweiligen Keilelemente kann zu dem aufeinander Zubewegen und voneinander Wegbewegen korrespondierend sein, also insbesondere schräg dazu, vorzugsweise orthogonal. Vorzugsweise ist die Bewegungsrichtung der Keilelemente in einer jeweiligen Ebene im Wesentlichen orthogonal zu der Längsachse. Insbesondere können die beiden Keilelemente um die Längsachse zueinander in dieser Ebene rotiert werden, um so die Keilelemente auseinander zu schieben oder aufeinander zu bewegen. Dadurch kann die Lageranordnung besonders kompakt sein. Alternativ kann auch nur das nicht rotationsfest mit dem Lagerelement verbundene Keilelement um die Längsachse rotiert werden, um so die Keilelemente auseinander zu schieben oder aufeinander zu bewegen.

Vorzugsweise ist die Verstellvorrichtung dabei so ausgebildet, dass sich die jeweiligen Keilelemente bei deren relativen Bewegung zueinander nicht oder nur unwesentlich über die Außenmaße der beiden Lagerelemente hinaus erstrecken. So muss auch kein zusätzlicher Bauraum für die Keilelemente und deren Bewegung vorgesehen werden. Insbesondere bei einer rotatorischen Bewegung der Keilelemente relativ zueinander um die Längsachse ist kein zusätzlicher Bauraum in radialer Richtung notwendig und die Lageranordnung kann besonders kompakt sein.

In weiterer vorteilhafter Ausgestaltung der Lageranordnung sind die beiden Keilelemente als Gleichtteile ausgebildet. Dadurch kann die Lageranordnung besonders kostengünstig sein, insbesondere durch Reduktion der Teilevarianz und mögliche Nutzung von Skaleneffekten bei Spritzgussteilen als Keilelementen. Vorzugsweise werden die beiden Keilelemente dabei um 180° zueinander gedreht aufeinander aufgelegt, insbesondere mit zueinander zugewandten und/oder miteinander kontaktierten jeweiligen Keilflächen.

In weiterer vorteilhafter Ausgestaltung der Lageranordnung ist es vorgesehen, dass die beiden Keilelemente an ihren Keilflächen gegeneinander verspannt sind, insbesondere mittels wenigstens eines Federelements. Dadurch werden die beiden Keilelemente aneinandergehalten bzw. an ihren Keilflächen weiter in Kontakt gehalten, auch wenn die Keilflächen so aneinander abgleiten, dass sich die beiden Keilelement aufeinander zubewegen sollen. Insbesondere kann damit auch ein Kontakt entgegen der Schwerkraft sichergestellt werden und die Lageranordnung bzw. die Verstellvorrichtung lageunabhängig funktionieren. Durch das Federelement wird dabei verhindert, dass die beiden Keilelemente voneinander abheben und so ungewollt ein Spiel in der Verstellvorrichtung entstehen kann. Das Federelement ist dabei ein besonders simples und kostengünstiges Element, um dies sicherzustellen. Das Federelement kann dafür sorgen, dass die Verzahnung des oberen Keilelements in die Verzahnung des Bremsring im gebremsten Zustand eingekuppelt ist und/oder eingekuppelt bleibt.

In weiterer vorteilhafter Ausgestaltung der Lageranordnung ist es vorgesehen, dass wenigstens eines der beiden Keilelemente als Ringelement ausgebildet ist, welches zum relativen Bewegen der Keilelemente zueinander um die Längsachse rotierbar angeordnet ist. Vorzugsweise sind dabei beide Keilelemente als Ringelemente ausgebildet und/oder um die Längsachse rotierbar befestigt. Dadurch kann die Lageranordnung besonders bauraumsparend sein und die Keilelemente können sich gleichmäßig in jeder Position besonders gut aneinander abstützen. Wenn beide Keilelemente als Ringelemente ausgebildet sind, sind diese vorzugsweise als Gleichteile ausgebildet, um die Kosten gering zu halten. Vorzugsweise ist dabei das andere der beiden Keilelemente rotationsfest zu einem der beiden Lagerelemente ausgebildet bzw. an diesem befestigt, um so die relative Rotation der beiden Keilelemente zueinander um die Längsachse zu vereinfachen. Aufgrund der Rotation der Keilelemente können die Keilflächen aneinander abgleiten und sich die Keilelemente aufeinander zu- oder wegbewegen. Vorzugsweise sind dabei beide Keilelemente an einem der Lagerelemente angebracht, wobei eines der beiden Keilelemente wenigstens entlang der axialen Richtung beweglich angeordnet ist. Vorzugsweise ist dabei nicht das gleiche Keilelement sowohl bezüglich der Längsachse rotierbar als auch entlang der Längsachse axial beweglich, um ein Spiel der Verstelleinrichtung gering zu halten und/oder um das Risiko einer Verkantung der beiden Keilelemente aneinander zu reduzieren. Das Ringelement bzw. die Ringelemente können dabei auch als Hubscheibe oder Hubscheiben bezeichnet werden. Durch das Abgleiten der Ringelemente aneinander kann ein Hub mittels der Verstellvorrichtung erzeugt werden.

Vorzugsweise weist wenigstens eines der beiden Keilelemente eine Vertiefung und/oder eine Durchgangsöffnung für einen Keilbereich bzw. die Keilfläche des anderen der beiden Keilelemente auf. In dieser Vertiefung und/ oder Durchgangsöffnung kann wenigstens ein Teil der Keilfläche bzw. des Keilbereichs des anderen Keilelements wenigstens teilweise aufgenommen werden, insbesondere in bestimmten Positionen relativ zueinander. Dadurch kann der Bewegungsspielraum der beiden Keilelemente relativ zueinander besonders groß sein, wodurch die Lageranordnung auch besonders kompakt sein kann. Beispielsweise kann ein Keilbereich eines Ringelements in einen Schlitz des anderen als Ringelement ausgebildeten Keilelements in einer gewissen rotatorischen Stellung der beiden Ringelemente zueinander hineingleiten, so dass sich die beiden Ringelemente besonders weit aufeinander zubewegen können. Die Vertiefung kann auch durch einen Keilbereich des jeweiligen Keilelements gebildet sein, welcher dann auch als Rampe ausgebildet sein kann.

In weiterer vorteilhafter Ausgestaltung der Lagerandordnung ist es vorgesehen, dass die beiden Keilelemente mittels einer gemeinsamen Führung an einem der beiden Lagerelemente befestigt sind. Durch eine gemeinsame Führung kann die Anzahl benötigter Teile besonders gering sein, wodurch die Lageranordnung besonders einfach zu montieren und zu warten sein kann. Darüber hinaus kann die Lageranordnung so besonders kostengünstig sein. Vorzugsweise ist die Führung dabei so ausgebildet, dass eines der beiden Keilelemente an dem Lagerelement, an welchem dieses mittels der Führung befestigt sind, rotationsfest aber axial entlang der Längsachse beweglich befestigt ist. Das andere der beiden Keilelemente kann dagegen beispielsweise in einem bestimmten Winkelbereich relativ zu dem Lagerelement, an welchem die Keilelemente befestigt sind, rotieren. So wird eine relative Bewegung der beiden Keilelemente zueinander zum Verändern deren Abstands ermöglicht. Der Abstand kann sich dabei beispielsweise auf einen Abstand jeweiliger Mittelpunkte der beiden Keilelemente beziehen. Ein Abstand ist beispielsweise nicht hinsichtlich einer Lücke oder Beabstandung zu verstehen, da die Keilelemente vorzugsweise innerhalb ihres gesamten Bewegungsbereichs mit den Keilflächen in Kontakt bleiben. Das rotierbar befestigte Keilelement ist dabei vorzugsweise in axialer Richtung entlang der Längsachse an dem Lagerelement fixiert, um ein Spiel in der Verstellvorrichtung zu reduzieren.

In weiterer vorteilhafter Ausgestaltung der Lageranordnung ist es vorgesehen, dass die Führung vorzugsweise eine Mehrzahl von über einem Umfang der als Ringelemente ausgebildeten Keilelemente angeordnete Führungsstifte umfasst und eines der beiden Keilelemente rotationsfest aber axial beweglich bezüglich der Längsachse an den Führungsstiften befestigt ist, insbesondere mittels jeweiligen in ihrem Querschnitt zu den Führungsstiften korrespondierenden Durchgangsöffnungen, und das andere der beiden Keilelemente rotierbar bezüglich der Längsachse an den Führungsstiften befestigt ist, insbesondere mittels jeweiligen Langlöchern. Dabei handelt es sich um eine konstruktiv einfache Führung, welche besonders robust sein kann, hohe Lasten aufnehmen kann und/oder einfach zu montieren sein kann. Vorzugsweise sind die Langlöcher korrespondierend zu den Führungsstiften und erlauben beispielsweise eine Rotation des einen der beiden Ringelemente um 40° bis 120° um die Längsachse, vorzugsweise 60° bis 80°, besonders bevorzugt ca. 75°. Vorzugsweise umfasst die Führung dabei wenigstens drei über den Umfang regelmäßig beabstandete Führungsstifte, vorzugsweise vier über den Umfang gleichmäßig voneinander beabstandete Führungsstifte. Dadurch kann eine statisch bestimmte Führung einfach zur Verfügung gestellt werden. Die Anzahl kann vorteilhafterweise zu einer Anzahl jeweiliger Keilflächen des jeweiligen Keilelements korrespondieren. Die Führungsstifte sind dabei simple Bauteile, insbesondere werden keine unterschiedlichen Führungselemente für die beiden Ringelemente benötigt. Die jeweiligen Langlöcher können dabei jeweils einen Anschlag an ihren jeweiligen Enden ausbilden, mittel welchem die rotatorische Bewegung des entsprechenden Ringelements einfach begrenzt werden kann. Die Führungsstifte können beispielsweise an einem insbesondere freien Ende einen Kopfbereich aufweisen, welcher ebenfalls einen Anschlag bildet, welcher die axiale Bewegung des axial entlang der Längsachse beweglichen Ringelements begrenzt. Das freie Ende kann dabei beispielsweise zu einem dem Lagerelement abgewandten Ende korrespondieren, an welchem die Führungsstifte montiert sind.

Vorzugsweise sind auch bei der Lageranordnung mit der Führung die beiden Ringelemente als Gleichteile ausgebildet. Beispielsweise kann jedes der Ringelemente sowohl zu dem Führungsstiften korrespondierende Durchgangsöffnung als auch Langlöcher umfassen. Je nach Anordnung der Ringelemente an den Führungsstiften sind diese dann um die Längsachse rotierbar und/oder axial beweglich und/oder in der Längsachse rotatorisch festgelegt. Vorzugsweise können dabei die jeweiligen Langlöcher gleichzeitig als Aufnahmebereich der Keilbereiche des anderen Ringelements in bestimmten Winkelstellungen dienen. So können auf zusätzliche Vertiefung und/oder Durchgangsöffnungen verzichtet werden, wodurch die jeweiligen Keilelemente und damit auch die Lageranordnung besonders kostengünstig sein kann.

Die beiden Keilelemente können beispielsweise als kostengünstige Kunststoffteile gespritzt werden. Dabei können die jeweiligen Langlöcher und/ oder Durchgangsöffnungen unmittelbar mit dem Spritzguss eingebracht werden, wodurch auf eine zusätzliche Nachbearbeitung vorzugsweise verzichtet werden kann.

In weiterer vorteilhafter Ausgestaltung der Lageranordnung umfasst die Verstellvorrichtung einen Aktuator, mittels welchem die Keilelemente relativ zueinander bewegbar sind. Mittels des Aktuators kann also auf eine manuelle Betätigung der Verstellvorrichtung verzichtet werden. Stattdessen kann die Betätigung beispielsweise durch ein Sensorsignal, beispielsweise eines Berührungssensors, und/oder durch eine Eingabevorrichtung, insbesondere einen Knopf umfassend, ausgelöst werden. Der Aktuator kann auch als Aktor bezeichnet werden. Gleichzeitig kann mittels des Aktuators noch eine weitere Übersetzung bereitgestellt werden, beispielsweise durch ein Getriebe eines Motors des Aktuators und/oder eine Verzahnung des Aktuators mit einem der beiden Keilelemente. Aufgrund der hohen so bereitstellbaren Übersetzung können dabei einfache Standardaktuatoren verwendet werden, wie beispielsweise Schrittmotoren oder Servomotoren. Gegebenenfalls kann der Aktuator auch mit einem Sensor und/oder einer Steuervorrichtung verbunden werden, um so eine automatische Modifizierung der Bremskraft, insbesondere der Reibkraft, zur Verfügung stellen zu können.

Beispielsweise kann der Aktuator einen Elektromotor umfassen oder aus einem Elektromotor gebildet sein, wobei auf einer Getriebeausgangswelle beispielsweise ein Zahnrad angeordnet sein kann, welches in Eingriff mit einem korrespondierenden Zahnbereich eines als Ringelement ausgebildeten Keilelements angeordnet ist und dieses Ringelement so um die Längsachse rotieren kann. Dadurch wird eine weitere Übersetzungsstufe zur Verfügung gestellt. Der Zahnbereich an dem Ringelement kann beispielsweise außenseitig oder innenseitig angeordnet sein. Bei einer außenseitigen Anordnung kann der Aktuator besonders einfach montiert werden und zudem auch besonders einfach gewartet werden, beispielsweise durch eine ebenfalls außenseitige Anordnung an einem der beiden Lagerelemente. Bei einem innenseitigen Zahnbereich kann der Aktuator ebenfalls innenseitig angeordnet sein, insbesondere radial innenseitig an einem der beiden Lagerelemente. Dadurch kann die Lageranordnung besonders kompakt sein. Innen und außen kann sich vorliegend beispielsweise auf eine radiale Richtung in Bezug auf die Längsachse beziehen. Vorzugsweise kann der Zahnbereich als komplett umlaufender Zahnkranz des Ringelements ausgebildet sein.

Werden Gleichteile als Ringelemente mit äußerer oder innerer Verzahnung benutzt, kann das Ringelement, welches nicht in Eingriff mit dem Aktuator steht, vorzugsweise einen Eingriff mit einer Kupplung und/oder einem Reibelement der Lageranordnung stehen. Dadurch kann dieses Ringelement bei einer relativen Bewegung der beiden Ringelemente zueinander in Eingriff gebracht werden bzw. der Eingriff getrennt werden, um so gleichzeitig die Reibkraft zu verstellen und die Bremsvorrichtung einzukuppeln oder auszukuppeln.

Beispielsweise kann die Verstellvorrichtung auch dazu ausgebildet sein, auf einem ersten Wegstreckenteil der Verstellung eine Vorspannkraft zum Erzeugen der Reibkraft einzustellen und auf einem weiteren Wegstreckenteil die Kupplungsvorrichtung von dem geöffneten in den geschlossenen Zustand und umgekehrt zu verstellen. Dann kann die Verstellvorrichtung vorteilhafterweise sowohl zum Einstellen der Betätigungskraft als auch zum Betätigen der Kupplungsvorrichtung genutzt werden.

In weiterer vorteilhafter Ausgestaltung der Lageranordnung ist es also vorgesehen, dass wenigstens eines der Keilelemente eine Verzahnung aufweist, welche mit einer korrespondierenden Verzahnung des Aktuators zum Bewegen dieses Keilelements in Eingriff steht.

Weitere Merkmale der Erfindung ergeben sich aus den Ansprüchen, den Ausführungsbeispielen sowie anhand der Zeichnungen. Die vorstehend in der Beschreibung genannten Merkmale und Merkmalskombinationen sowie die nachfolgend in den Ausführungsbeispielen genannten Merkmale und Merkmalskombinationen sind nicht nur in der jeweils angegebenen Kombination, sondern auch in anderen Kombinationen verwendbar, ohne den Rahmen der Erfindung zu verlassen.

Dabei zeigt:
- Fig. 1: in einer schematischen Ansicht ein Schema einer Lageranordnung mit einer Verstellvorrichtung mit zwei aneinander an ihren jeweiligen Keilflächen anliegenden Keilelementen;
- Fig. 2 und 3: in schematischen Seitenansichten einen Teil der Lageranordnung gemäß Fig. 1;
- Fig. 4: in einer schematischen Perspektivansicht von der Lageranordnung gemäß Fig. 2 und 3 die jeweiligen Keilflächen der Keilelemente, wobei die Darstellung teilweise geschnitten ist; und
- Fig. 5: in einer schematischen Schnittansicht eine weitere Ausführungsform der Lageranordnung gemäß Fig. 1.

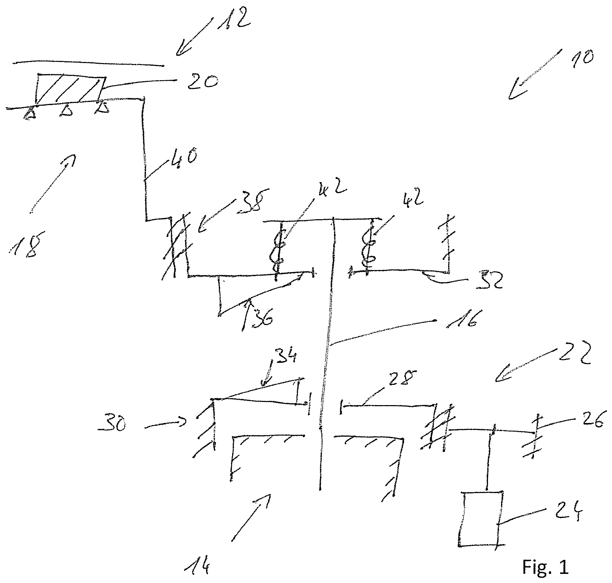

Fig. 1 zeigt ein Schema einer Lageranordnung 10 mit einem ersten Lagerelement 12, welches als Stator ausgebildet ist und mit einem zweiten Lagerelement 14, welches als Rotor ausgebildet ist. Die beiden Lagerelemente 12 und 14 können relativ zueinander um die Längsachse 16 rotieren. Die Lageranordnung 10 ist beispielsweise besonders geeignet dafür, einen Tragarm drehbar zu befestigen, an welchem jeweilige medizinische Geräte gehalten werden können.

Die Lageranordnung 10 umfasst dabei eine Bremsvorrichtung 18, welche die relative Rotation der beiden Lagerelemente 12, 14 zueinander mittels einer durch die Bremsvorrichtung 18 erzeugten Reibkraft hemmt bzw. hemmen kann. Dafür wird ein Reibelement 20 an eine Fläche des ersten Lagerelements 12 gepresst, wodurch hier bei einer Rotation der beiden Lagerelemente 12, 14 eine Reibkraft entsteht, welche der Rotation entgegenwirkt. Dafür ist das Reibelement 20 rotationsfest mit dem zweiten Lagerelement 14 verbunden.

Vorliegend ist dabei die Reibkraft mittels einer Verstellvorrichtung 22 einstellbar. Zu diesem Zweck umfasst die Verstellvorrichtung 22 ein Aktuator 24, welcher mittels eines Zahnrads 26 mit einem ersten Keilelement 28 in Eingriff steht. Dafür umfasst das erste Keilelement 28 eine radial äußere Verzahnung 30, welche mit dem Zahnrad 26 in Eingriff steht. Dadurch erfolgt eine Übersetzung von der Ausgangswelle des Aktuators 24 über das Zahnrad 26 zu dem Keilelement 28. Das Keilelement 28 ist dabei vorliegend als Ringelement bzw. Scheibe ausgebildet, welche an ihrer an einem anderen Keilelement 32 zugewandten Seite mehrere Keilflächen 34 aufweist. Das gegenüberliegend angeordnete zweite Keilelement 32 ist dabei als Gleichteil und somit identisch mit dem ersten Keilelement 28 ausgebildet und ebenfalls mit seinen jeweiligen Keilflächen 36 dem ersten Keilelement 28 zugewandt angeordnet. Das Keilelement 32 ist lediglich bezüglich dem Keilelement 28 um 180° gedreht angeordnet. Obwohl vorliegend voneinander in Fig. 1 beabstandet dargestellt, liegen die beiden Keilflächen 34, 36 aneinander an. Die Keilflächen 34, 36 sind beispielsweise als mehrere Keilbereiche über den Umfang der beiden als Scheiben bzw. Ringelemente ausgebildeten Keilelemente 28, 32 angeordnet und dabei auch gekrümmt dem Umfang folgend ausgebildet. Insbesondere können auf jeder der beiden Keilelemente 28, 32 vier gleichmäßig über den Umfang verteilte Keilflächen 34, 36 angeordnet sein, welche jeweils identisch sein können. Die Keilelemente 28, 32 weisen dann beispielsweise eine vierfache Rotationssymmetrie bezüglich der Längsachse 16 auf.

Weiterhin ist das in der Fig. 1 als oberes Keilelement 32 dargestellte Keilelement 32 über seine Verzahnung 38 mit einem Bremsring 40 verbunden, an welchem das Reibelement 20 befestigt ist. Das obere Keilelement 32 ist dabei rotationsfest mit dem Rotor bzw. dem zweiten Lagerelement 14 verbunden, so dass das Reibelement 20 ebenfalls mit diesem mit dreht.

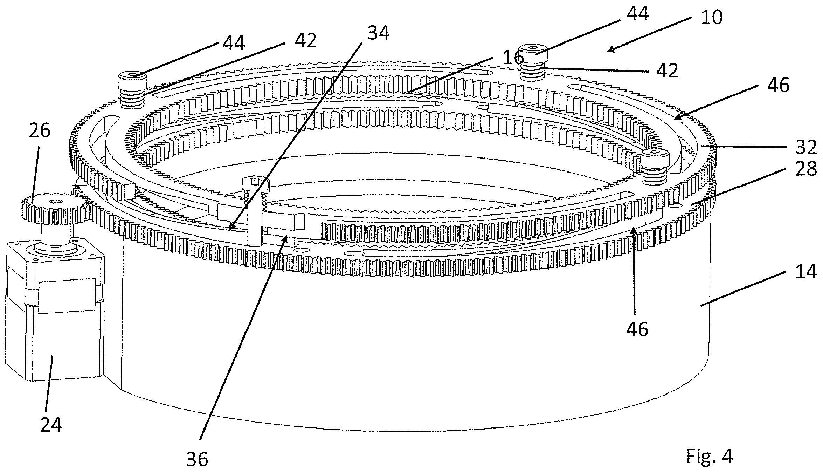

Zur Modifizierung der Bremskraft kann mittels des Aktuators 24 das in der Fig. 1 untere dargestellte Keilelement 28 um die Längsachse 16 rotiert werden, wodurch die beiden Keilflächen 34, 36 aneinander abgleiten. Dies ist beispielsweise auch besonders gut in der schematischen Perspektivansicht dieser Keilflächen 34, 36 in Fig. 4 zu erkennen. Bei einer Drehung bzw. Rotation des Keilelements 28 um die Längsachse 16 in einer Richtung bewegen sich deswegen die beiden Keilelemente 28, 32 aufeinander zu, und bei einer Drehung in eine entgegengesetzte Richtung bewegen sich die beiden Keilelemente 28, 32 voneinander weg. Entsprechend kann so auch der Bremsring 40 und das Reibelement 20 entlang der Längsachse 16 axial mitbewegt. Die Verzahnung 38 kann entsprechend zur Übertragung der Bewegung ausgebildet sein. Bei einer Bewegung der beiden Keilelemente 28, 32 voneinander weg wird so das Reibelement 20 in der Fig. 1 nach oben bewegt und damit stärker gegen das erste Lagerelement 12 bzw. den Stator gepresst. Durch diese Erhöhung des Anpressdruckes resultiert eine höhere Reibkraft bei einer relativen Rotation der beiden Lagerelemente 12, 14 zueinander um die Längsachse 16. Umgekehrt wird bei einem Aufeinanderzubewegen der beiden Keilelemente 28, 32 der Anpressdruck des Reibelements 20 gegen das erste Lagerelement 12 verringert, womit eine geringere Reibkraft eingestellt werden kann. Daraus resultiert dann ein geringeres Bremsmoment.

Um für ein besonders geringes Spiel bei der Verstellvorrichtung 22 zu sorgen und/oder eine Bewegung der beiden Keilelemente 28, 32 unabhängig von einer räumlichen Lage der Lageranordnung 10 zu ermöglichen, ist das obere Keilelement 32 mittels jeweiliger Federelemente 42 axial entlang der Längsachse 16 gegen das untere Keilelement 28 verspannt. Bei den Federelementen 42 handelt es sich beispielsweise um Druckfedern. Dadurch stehen die beiden Keilflächen 34, 36 zuverlässig in Kontakt zueinander. Insbesondere wenn die beiden Keilelemente 32, 28 aufeinander zubewegt werden sollen, können die Druckfedern 42 die beiden Keilelemente 28, 32 zuverlässig in Kontakt halten.

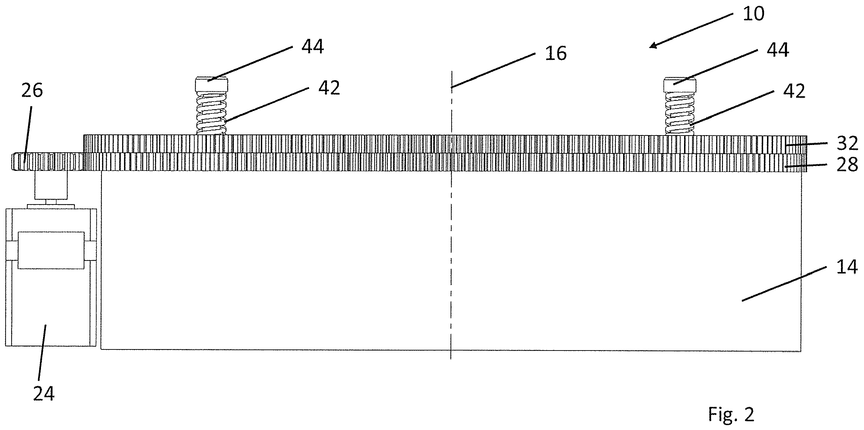

Die jeweiligen Federelemente 42 sind dabei mit ihrer Mittelachse über die jeweiligen Führungsstifte 44 angeordnet, mittels welchen die beiden Keilelemente 28, 32 geführt sind. Diese Führung ist insbesondere in den Figuren 2 bis 4 gut zu erkennen. Dabei sind die jeweiligen Führungsstifte 44 gleichmäßig über den Umfang der beiden ringförmigen Keilelemente 28, 32 verteilt. Bei dem oberen Keilelement 32 sind dabei die Führungsstifte 44 in jeweiligen Durchgangsöffnungen aufgenommen, deren Durchmesser zu dem Durchmesser der Führungsstifte 44 korrespondiert. Dadurch ist das obere Keilelement 32 bezüglich der Längsachse 16 rotationsfest mit dem Rotor bzw. dem zweiten Lagerelement 14 verbunden, zumindest in dem in Fig. 2 bis 4 gezeigten Ausführungsbeispiel. Gleichzeitig kann aber das obere Keilelement 32 entlang der Längsachse 16 entlang den Führungsstiften 44 axial bewegt werden, um die beiden Keilelemente 28, 32 aufeinander zu- und voneinander wegbewegen zu können.

Bei dem unteren Keilelement 28 sind dagegen die Führungsstifte 44 in jeweiligen Langlöchern 46 aufgenommen, so dass das untere Ring- bzw. Keilelement 28 um die Längsachse 16 in einem durch die jeweiligen Langlöcher 46 vorgegebenen Bewegungsbereich sowohl relativ gegenüber dem zweiten Lagerelement 14 als auch dem oberen Keilelement 32 rotiert werden kann. Beispielsweise beträgt die Rotationsbereich 75°, bevor die jeweiligen Langlöcher 46 mit ihren jeweiligen Endbereichen an den Führungsstiften 44 anschlagen.

In Fig. 2 sind dabei die beiden Keilelemente 28, 32 in einer Stellung gezeigt, in welcher diese am weitesten aufeinander zubewegt sind. Die beiden Keilelemente 28, 32 liegen dabei im Wesentlichen plan aufeinander auf. Damit ist die geringmöglichste Anpresskraft des Reibelements 20 an dem ersten Lagerelement 12 eingestellt. Beispielsweise kann das Reibelement 20 hierbei überhaupt nicht in Kontakt mit dem ersten Lagerelement 12 stehen, so dass die beiden Lagerelemente 12, 14 im Wesentlichen reibungsfrei zueinander um die Längsachse 16 rotiert werden können.

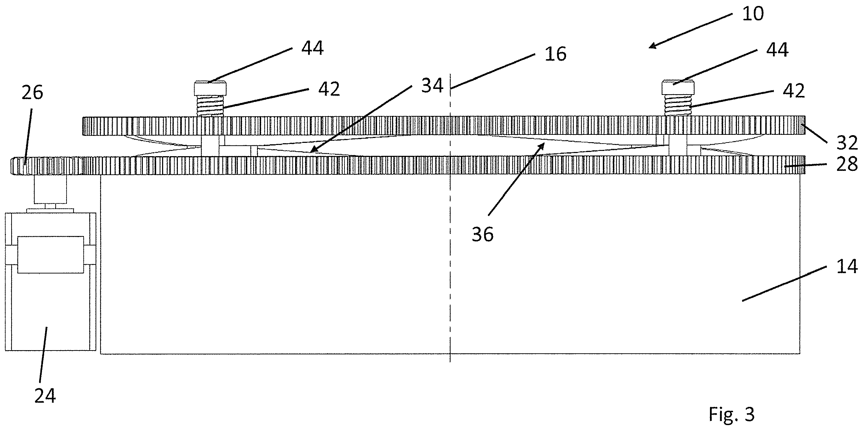

In Fig. 3 sind dagegen die beiden Keilelemente 28, 32 in einer Stellung gezeigt, in welcher diese am weitesten voneinander abgehoben und damit auseinanderbewegt sind. Die beiden Keilelemente 28, 32 können entsprechend auch als Hubscheiben bezeichnet werden, da sie eine relative Rotation zueinander um die Längsachse 16 in einen Hub umsetzen. Dabei sind die beiden Keilflächen 34, 36 so aneinander abgeglitten, dass diese im Wesentlichen nur noch mit ihren höchsten Teilbereichen aufeinander aufliegen. Dies ist insbesondere in der teilweise geschnittenen Darstellung in Fig. 4 zu erkennen. Entsprechend ist auch das Reibelement 20, wie dies dann beispielsweise in der Ansicht gemäß Fig. 1 zu erkennen wäre, weit nach oben gehoben, sodass dieses maximal gegen das erste Lagerelement 12 gepresst wird. Diese Anpresskraft kann dabei im Wesentlichen so hoch sein, dass die beiden Lagerelemente 12, 14 im Wesentlichen rotatorisch um die Längsachse 16 zueinander fixiert sind.

Durch den Aufbau der Lageranordnung 10 ergeben sich drei hintereinander geschaltete Übersetzungsstufen. Die erste Übersetzungsstufe ist eine Getriebeübersetzung des Aktuators 24, beispielsweise des darin verbauten Elektromotors. Die zweite Übersetzungsstufe kann beispielsweise zwischen dem antreibenden Zahnrad 26 und dem unteren Keilelement 28, welches auch als untere Hubscheibe bezeichnet werden kann, vorgesehen sein. Eine dritte Übersetzungsstufe und damit Kraftverstärkung ergibt sich durch die aufeinander abgleitenden Keilflächen 34, 36. Eine Gesamtübersetzung ergibt sich beispielsweise durch die Multiplikation der drei einzelnen Übersetzungen. Die axiale Hubkraft zum Einstellen der Bremskraft oder zum alternativen oder zusätzlichen Auskoppeln aus der Verzahnung 38 mit dem Bremsring 40 berechnet sich beispielsweise zu F_{H} = M_{GA} * i_{R} * i_{H} / r_{Keil}, wobei M_{GA} das Getriebeausgangsmoment ist, i_{R} das Übersetzungsverhältnis zwischen Zahnrad und unterer Hubscheibe, i_{H} die Kraftverstärkung der Keilfläche und rₖₑᵢₗ der Radius der Kreisfläche. Reibungseinflüsse sind bei dieser Gleichung nicht berücksichtigt. Die Verstärkung kann dabei derart hoch sein, dass beispielsweise auch die obere Hubscheibe bzw. das obere Keilelement 32 unmittelbar zur Erzeugung der Reibkraft bzw. Bremskraft benutzt werden kann, ohne das zusätzliche Vorsehen des Reibelements 20. Dafür können beispielsweise die beiden Keilelemente 28, 32 aus Metall ausgebildet sein.

Wie insbesondere in Fig. 4 zu erkennen ist, sind die beiden Keilflächen 34, 36 zur Hälfte unterhalb und zur Hälfte oberhalb der planaren Oberfläche, welche dem jeweils anderen Keilelement zugewandt ist, des restlichen Keilelements 28, 32 bzw. deren jeweilige Ringbereiche ausgebildet. Dadurch kann die Keilfläche 36 mit ihrem hervorstehenden Teilbereich in den unterhalb der planaren Ebene des unteren Keilelements 28 liegenden Teilbereichs der Keilfläche 36 abgleiten, so dass die beiden Keilelemente 28, 32 im Wesentlichen mit ihren Ringteilbereichen aneinander anliegen können. Dadurch kann die Verstellvorrichtung 22 besonders bauraumsparend sein.

Alternativ oder zusätzlich kann die Verstellvorrichtung 22 auch dazu verwendet werden, die Bremsvorrichtung 18 aus einem Kraftpfad zwischen den beiden Lagerelementen 12, 14 auszukuppeln. Dafür kann die Verzahnung 38 dazu ausgebildet sein, bei einer axialen Verschiebung ihren Eingriff zu lösen.

Ein Vorgang des Auskuppelns der vorliegenden Bremsvorrichtung 18, insbesondere der in Fig. 1 gezeigten Bremsvorrichtung 18, kann dabei wie folgt erfolgen: Der Aktuator 24 wird bestromt und bewirkt an seiner Antriebsachse eine Drehbewegung. Ein gegebenenfalls vorgeschaltetes Motorgetriebe verstärkt das Drehmoment entsprechend. Auf der Getriebeausgangswelle ist ein Zahnrad 26 befestigt, dessen Zähne sich im Eingriff mit der Außenverzahnung 30 der unteren Hubscheibe 28 befinden. Da der Durchmesser des antreibenden Zahnrads 26 wesentlich kleiner ist als der Durchmesser der Hubscheibe 28, wird das Drehmoment nochmals verstärkt und die Drehzahl dabei reduziert. Die Keilflächen 34 der unteren Hubscheibe 28 sind dabei derart angeordnet, dass diese Keilflächen 34 nach oben weisen. In eine Oberseite eines Lageraußenrings sind die als Passschrauben ausgebildeten Führungsstifte 44 eingebracht, welche der Führung der beiden Hubscheiben 28, 32 dienen. Die obere Hubscheibe 32 ist dabei über die Passschrauben so geführt, dass sie rotationsfest gelagert jedoch axial verschiebbar ist. Zwischen der Oberseite der oberen Hubscheibe 32 und dem Schraubenkopf der Passstifte 44 bzw. Führungsstifte 44 wirken dabei die Druckfedern 42, welche die obere Hubscheibe 32 an die untere Hubscheibe 28 pressen. Im eingekoppelten Zustand ist die Verzahnung 38 der oberen Hubscheibe 32 im Eingriff mit der entsprechenden Verzahnung des Bremsrings 40. Wird die untere Hubscheibe 28 durch den Aktuator 24 gedreht, gleiten die Keilflächen 34, 36 aufeinander ab. Die obere Hubscheibe 32 wird gegen die Federkraft der Druckfedern 42 nach oben bewegt und gleitet aus der Verzahnung des Bremsrings 40 heraus. Hier ist also die Bremswirkung aufgehoben, womit die Verstellvorrichtung 22 als Kupplung wirkt.

Im Gegensatz zum vorher geschilderten Ausführungsbeispiel, bei welchem die Verstellvorrichtung 22 lediglich zur Einstellung der bei der Drehung der beiden Lagerelemente 12, 14 zueinander erzeugten Reibkraft dient, kann hier also die Verzahnung 38 mittels der Verstellvorrichtung 22 gelöst werden und damit die Bremsvorrichtung 18 ausgekuppelt werden. Die Bremswirkung ist also aufgehoben, da das zweite Lagerelement 14 vom Bremsring 40 entkuppelt ist.

Bei diesem Ausführungsbeispiel kann die Vorspannkraft des Reibelements 20 separat von der Verstellvorrichtung 22 bereitgestellt werden, beispielsweise durch ein Spannelement und/oder eine weitere Verstellvorrichtung mit Keilelementen.

Soll die Bremswirkung wiederhergestellt werden, verdreht der Aktuator 24 die untere Hubscheibe 28 um den gleichen Winkel zurück. Die obere Hubscheibe 32 wird dabei durch die Federkraft entsprechend wieder nach unten gedrückt. Die Verzahnung 38 der oberen Hubscheibe 32 kann dabei auf der Verzahnung des Bremsrings 40 aufliegen und rastet dann erst bei einer aber nur sehr geringen Bewegung eines an der Lageranordnung 10 befestigten Tragarmsystems und damit relativen Drehung der Lagerelement 12, 14 zueinander selbstständig wieder ein. Dadurch ist die Bremswirkung wiederhergestellt.

Das Zahnrad 26 ist vorzugsweise ein Katalogartikel. Bei dem Aktuator 24 kann z.B. ein Schrittmotor eingesetzt werden. Die Anzahl der Schritte und die Drehrichtung kann von einer Steuerelektronik, beispielsweise einem integrierten Schaltkreis und/oder einem Mikroprozessor, vorgegeben werden. Hierdurch kann dann softwareseitig auch die Dauer der Freistellung der Bremswirkung vorgegeben werden. Alternativ kann auch ein Gleichstrommotor verwendet werden, welcher durch Signalgeber in die jeweiligen Endlagen gesteuert wird.

Die Lageranordnung 10 nutzt ein Wirkprinzip, welches den Einsatz von günstigen, einfach zu beschaffenden elektrischen Aktuatoren 24, vorzugsweise kleine Getriebemotoren, und den Einsatz von kostengünstig herstellbaren Komponenten vorsieht, insbesondere von einer hohen Anzahl von Gleichteilen. Insbesondere um einfache und günstige Getriebemotoren einsetzen zu können, sollte deren Antriebsmoment verstärkt werden. Dies erfolgt vorliegend beispielsweise mittels der drei Übersetzungsstufen, wobei zwei davon in einem zweifach verwendeten Spritzgussteil, nämlich den Keilelementen 28, 32 der Verstellvorrichtung 22, integriert sind. Die Lageranordnung 10 weist einen hohen Grad von Funktionsintegration in wenigen Bauteilen auf. Der Hub jeweiliger Kupplungsscheiben muss nicht mehr durch eine magnetische Krafteinwirkung einer stromdurchflossenen Spule erreicht werden. Auf die aufwändige Herstellung einer solchen Spule kann verzichtet werden. Ein Ein- und Auskuppeln der Kupplungsscheibe, welche hier dem Bremsring 40 und/oder der oberen Hubscheibe 32 entsprechen kann, kann mit einem einfachen Elektromotor realisiert werden. Im Gegensatz dazu muss bei einer herkömmlichen Kupplung mit einem Elektromagnet bezüglich eines großen Luftspalts aufgrund der für die Kupplungsfunktion notwendigen Distanz ausgelegt werden, wodurch ein starker und damit großer Elektromagnet notwendig ist.

Alternativ kann aber auch die Verstellvorrichtung 22 benutzt werden, um direkt die Vorspannung zur Erzeugung der Reibkraft zur Hemmung der Bewegung der beiden Lagerelemente 12, 14 der Lageranordnung 10 relativ zueinander zu erreichen.

Die beiden Keilflächen 34, 36 können beispielsweise, wie vorliegend, auch als Rampen ausgebildet sein. Vorliegend ist der Aktuator 24 außenseitig von den Lagerelementen 12, 14 angeordnet. Dadurch ist dieser leicht für eine Wartung zugänglich und hinsichtlich seiner Größe nicht beschränkt. Die jeweiligen Verzahnungen der beiden Keilelemente 28, 32 könnten jedoch auch radial innenseitig angeordnet sein und damit der Aktuator 24 in einem radial inneren Hohlraum eines der beiden Lagerelemente 12, 14. Damit kann die Lageranordnung 10 besonders klein sein und der Aktuator 24 ist geschützt.

Die Lageranordnung 10 ist gerade bei medizinischen Tragarmsystemen von Vorteil, da so bei baugleichen Lagerungen unterschiedliche Bremskräfte eingestellt werden können. Dadurch kann beispielsweise auch bewirkt werden, dass sich zunächst eine Lageranordnung mit einem kürzeren Hebelarm aufgrund eines kürzeren Tragarms in Bewegung setzt und erst anschließend eine Lageranordnung mit einem längeren Tragarm. So kann beispielsweise erreicht werden, dass bei einem zweigliedrigen Tragarmsystem mit einem langen Tragarm, welcher an der Decke eines OP-Saals befestigt ist, und einem kurzen Tragarm, welcher eine Feinjustierung unmittelbar in der Nähe des Benutzers ermöglicht, zunächst dieser kürzere Tragarm verstellt werden kann, ohne dass sich der längere Tragarm an der Decke bewegt. Zudem kann eine bereitgestellte Bremskraft bzw. Reibkraft, welche die Bewegung der beiden Lagerelemente 12, 14 relativ zueinander hemmt, nach Kundenwünschen eingestellt werden. Dabei ist die Lageranordnung 10 insgesamt kostengünstig und robust.

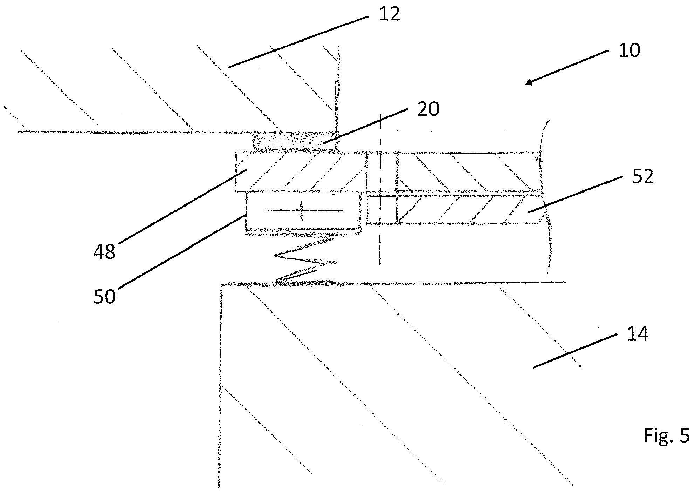

Fig. 5 zeigt in einer schematischen ausschnittsweisen Schnittdarstellung eine weitere Ausführungsform der Lageranordnung 10, bei welcher statt eines Bremsrings 40 eine Bremsscheibe 48 eingesetzt wurde, welche auf einen Lagerring wirkt, der nicht mit der Verstellvorrichtung 22 verbunden ist. Entsprechend kann das Reibelement 20 hier als Bremsbelag auf einer Bremsscheibe 48 ausgebildet sein. Dieses kann beispielsweise von dem zweiten Lagerelement 14 mittels eines Wälzlagers 50 rotatorisch entkoppelt sein und ebenfalls mittels eines oberen Keilelements bzw. der Hubscheibe 52 an das erste Lagerelement 12 gepresst werden.

### Bezugszeichenliste

- 10: Lageranordnung
- 12: erstes Lagerelement
- 14: zweites Lagerelement
- 16: Längsachse
- 18: Bremsvorrichtung
- 20: Reibelement
- 22: Verstellvorrichtung
- 24: Aktuator
- 26: Zahnrad
- 28: unteres Keilelement
- 30: Verzahnung
- 32: oberes Keilelement
- 34: Keilfläche
- 36: Keilfläche
- 38: Verzahnung
- 40: Bremsring
- 42: Federelement
- 44: Führungsstifte
- 46: Langloch
- 48: Bremsscheibe
- 50: Wälzlager
- 52: Hubscheibe

## Patentansprüche

1. Lageranordnung (10) mit wenigstens einem ersten Lagerelement (12) und einem zweiten Lagerelement (14), welche entlang einer gemeinsamen Längsachse (16) relativ rotierbar zueinander miteinander verbunden sind, wobei die Lageranordnung (10) eine Bremsvorrichtung (18) umfasst, welche die relative Rotation der beiden Lagerelemente (12, 14) zueinander mittels einer durch die Bremsvorrichtung (18) erzeugten Reibkraft hemmt, und
eine Verstellvorrichtung (22), mittels welcher die bei der relativen Rotation der beiden Lagerelemente (12, 14) zueinander wirkende Bremskraft modifizierbar ist,
wobei die Verstellvorrichtung (22) wenigstens zwei aneinander an ihrer jeweiligen Keilfläche (34, 36) anliegende Keilelemente (28, 32) umfasst, welche zum Modifizieren der Bremskraft aneinander abgleitend relativ zueinander bewegbar sind.

2. Lageranordnung (10) nach Anspruch 1, wobei
die Bremsvorrichtung (18) eine erste Reibfläche aufweist, welche mit dem ersten Lagerelement (12) um die Längsachse (16) rotationsfest mitrotiert und eine zweite Reibfläche, welche mit dem zweiten Lagerelement (14) um die Längsachse (16) rotationsfest mitrotiert,
wobei die beiden Reibflächen gegeneinander mit einer Vorspannkraft verspannt sind und bei der relativen Rotation der beiden Lagerelemente (12, 14) zueinander aneinander unter Erzeugung der Reibkraft abgleiten,
wobei die Verstellvorrichtung (22) dazu ausgebildet ist, diese Vorspannkraft durch Bewegen der Keilelemente (28, 32) relativ zueinander zum Einstellen der Reibkraft einzustellen und somit die Bremskraft zu modifizieren.

3. Lageranordnung (10) nach Anspruch 1 oder 2, wobei
die Lageranordnung (10) eine Kupplungsvorrichtung, welche zwischen einem offenen Zustand und einem geschlossenen Zustand verstellbar ist, umfasst,
wobei in dem geschlossenen Zustand die Bremsvorrichtung (18) eingekuppelt ist, wodurch bei einer Rotation der beiden Lagerelemente (12, 14) relativ zueinander diese Rotation durch die mittels der Bremsvorrichtung (18) erzeugte Reibkraft gehemmt ist, und
wobei in dem offenen Zustand die Bremsvorrichtung (18) ausgekuppelt ist, wodurch die beiden Lagerelemente (12, 14) frei von einer Hemmung durch die Bremsvorrichtung (18) relativ zueinander rotierbar sind, und
wobei die Verstellvorrichtung (22) dazu ausgebildet ist, durch Bewegen der Keilelemente (28, 32) relativ zueinander die Kupplungsvorrichtung zwischen ihrem offenen Zustand und ihrem geschlossenen Zustand zu verstellen und somit die Bremskraft zu modifizieren.

4. Lageranordnung (10) nach einem der vorhergehenden Ansprüche, wobei die Verstellvorrichtung (22) so ausgebildet ist, dass durch das relative Bewegen der Keilelemente (28, 32) zueinander sich diese in einer Richtung im Wesentlichen parallel zu der Längsachse (16) wahlweise aufeinander zubewegen oder voneinander wegbewegen.

5. Lageranordnung (10) nach einem der vorhergehenden Ansprüche, wobei die beiden Keilelemente (28, 32) als Gleichteile ausgebildet sind.

6. Lageranordnung (10) nach einem der vorhergehenden Ansprüche, wobei die beiden Keilelemente (28, 32) an ihren Keilflächen (34, 36) gegeneinander verspannt sind, insbesondere mittels wenigstens eines Federelements (42).

7. Lageranordnung (10) nach einem der vorhergehenden Ansprüche, wobei wenigstens eines der beiden Keilelemente (28, 32) als Ringelement ausgebildet ist, welches zum relativen Bewegen der Keilelemente (28, 32) zueinander um die Längsachse (16) rotierbar angeordnet ist.

8. Lageranordnung (10) nach einem der vorhergehenden Ansprüche, wobei die beiden Keilelemente (28, 32) mittels einer gemeinsamen Führung an einem der beiden Lagerelemente (12) befestigt sind,
wobei die Führung vorzugsweise eine Mehrzahl von über einen Umfang der als Ringelemente ausgebildeten Keilelemente (28, 32) angeordneten Führungsstiften (44) umfasst und eines der beiden Keilelemente (14) rotationsfest aber axial beweglich bezüglich der Längsachse (16) an den Führungsstiften (44) befestigt ist, insbesondere mittels jeweiligen mit ihrem Querschnitt zu den Führungsstiften (44) korrespondierenden Durchgangsöffnungen, und das andere der beiden Keilelemente (14) rotierbar bezüglich der Längsachse (16) an den Führungsstiften (44) befestigt ist, insbesondere mittels jeweiligen Langlöchern (46).

9. Lageranordnung (10) nach einem der vorhergehenden Ansprüche, wobei die Verstellvorrichtung (22) einen Aktuator (24) umfasst, mittels welchem die beiden Keilelemente (28, 32) relativ zueinander bewegbar sind.

10. Lageranordnung (10) nach einem der vorhergehenden Ansprüche, wobei wenigstens eines der Keilelemente (28, 32) eine Verzahnung (30) aufweist, welche mit einer korrespondierenden Verzahnung des Aktuators (24) zum Bewegen dieses Keilelements (28) in Eingriff steht.
